# EUROPÄISCHE PATENTSCHRIFT

(11) **EP 0 452 253 B1**
(45) Veröffentlichungstag und Bekanntmachung des Hinweises auf die Patenterteilung: **06.10.1993**
(21) Anmeldenummer: 91810155.1
(22) Anmeldetag: 08.03.1991
(51) Int. Cl.: A61F 2/36

(54) **Femurkopfprothese**
Femoral head prosthesis
Prothèse de la tête fémorale

(30) Priorität: 10.04.1990 CH 1212/90
(43) Veröffentlichungstag der Anmeldung: 16.10.1991
(73) Patentinhaber: SULZER Medizinaltechnik AG, 8404 Winterthur (CH)
(72) Erfinder: Willert, Hans-Georg, Prof. Dr. med., W-3400 Göttingen (DE); Koch, Rudolf, CH-8267 Berlingen (CH)
(74) Vertreter: Hammer, Bruno, Dr.

(56) Entgegenhaltungen:
- EP-A- 0 308 297
- EP-A- 0 354 142
- WO-A-89/01321
- FR-A- 2 425 237
- FR-A- 2 549 717

## Beschreibung

Die Erfindung betrifft eine Femurkopfprothese mit einem sich von distal nach proximal allseitig konisch erweiternden Verankerungsschaft, der mindestens einen die ganze Schaftdicke durchsetzenden, im wesentlichen in Längsrichtung verlaufenden schmalen Schlitz aufweist.

Eine Prothese der vorstehend genannten Art ist beispielsweise aus der FR-A-25 49 717 bekannt. Vorzugsweise parallel zu den Aussenflächen des Prothesenschaftes verlaufende schmale Längsschlitze durch den ganzen Schaft hindurch sollen bei dieser bekannten Konstruktion die Elastizität des, vorwiegend aus Metall gefertigten, Schaftes erhöhen und beim Implantieren des Schaftes einerseits übergrosse Druckspitzen verhindern und die Gefahr einer Knochensprengung vermindern, und andererseits durch eine grosse Elastizität weiten Bereichen ein Anpassen des Schaftes an Krümmungen und Unebenheiten des Femurhohlraumes ermöglichen und damit ein relativ grossflächiges Anliegen am Knochen gewährleisten.

Wie bei den bisherigen Schaftkonstruktionen üblich, müssen jedoch auch bei dieser bekannten Prothese durch Biegebelastungen auf den Kugelkopf hervorgerufene Kräfte, vor allem bei Be- und Entlastungen des Schaftes wechselnde Scher- und Torsionskräfte im distalen Bereich durch die "Grenzfläche" Knochen/Prothese hindurch übertragen bzw. von dieser aufgenommen werden.

Aufgabe der Erfindung ist es nun, eine Prothese zu schaffen, bei der, wie bei der vorstehend diskutieren Konstruktion, die Elastizität des Schaftes im distalen Bereich derjenigen des Knochens anzunähern, bei der jedoch der Uebergangsbereich von der Prothese auf den Knochen bzw. ein Knochenzementbett mindestens weitgehend von Scher- und Torsionskräften entlastet ist.

Diese Aufgabe wird nach der Erfindung dadurch gelöst, dass der Schlitz U-förmig im distalen Bereich des Schaftes angeordnet ist, wobei die freien Enden der Schenkel des U-förmigen Schlitzes nach proximal gerichtet sind.

Die U-Form des Schlitzes erlaubt, dem inneren Kern des Schaftes Kräften aufgrund von Biegemomenten auf den Gelenkkopf weitgehend nachzugeben, ohne dass dabei wesentliche Scher- und Torsionsbelastungen am Uebergangsbereich Prothese Knochen auftreten.

Relativ zu den Aussenschenkeln der U-Form kann sich der Kern dabei sowohl in Richtung medial/lateral als auch ventral/dorsal bewegen, sowie um seine Längsachse Torsionsbewegungen ausführen.

Soll der Randbereich über die ganze Höhe der U-Form eine annähernd konstante Biegespannung aufweisen, so verlaufen die sich in Längsrichtung erstreckenden Schenkel der U-Form zweckmässigerweise parallel zur Schaftachse. Nach proximal zunehmende Biegespannung im Randbereich resultiert, wenn die in Längsrichtung verlaufenden Schenkel der U-Form parallel zur Aussenform der Prothese liegen.

Unterschiedliche Steifigkeiten des Schaftes medial und lateral seiner Mittelachse lassen sich erzielen, wenn der lateral der Schaftachse verlaufende Schenkel kürzer ist als der mediale Schenkel. Dadurch wird eine zu starke Auslenkung des Kerns nach lateral vermieden, die zu seinem Anliegen an den lateralen U-Schenkel führen könnte. Eine Berührung beider sollte jedoch möglichst vermieden werden, da die Relativbewegungen anderenfalls unerwünschten Metallabrieb erzeugen.

Um dem Kern nach lateral ein weitgehendes Ausweichen zu ermöglichen, kann der laterale Schenkel der U-Form schliesslich breiter sein als der mediale, wobei seine Breite, ausgehend von einer gleichen Breite am proximalen Ende, von proximal nach distal stetig zunimmt.

Im folgenden wird die Erfindung anhand von Ausführungsbeispielen im Zusammenhang mit der Zeichnung näher erläutert.
- Fig. 1: zeigt eine Ansicht des distalen Endes einer ersten Ausführungsform des neuen Schaftes;
- Fig. 2 und 3: sind Schnitte II-II bzw. III-III von Fig. 1;
- Fig. 4 bis 6: geben in gleicher Darstellung wie Fig. 1 weitere Ausführungsbeispiele der Erfindung wieder.

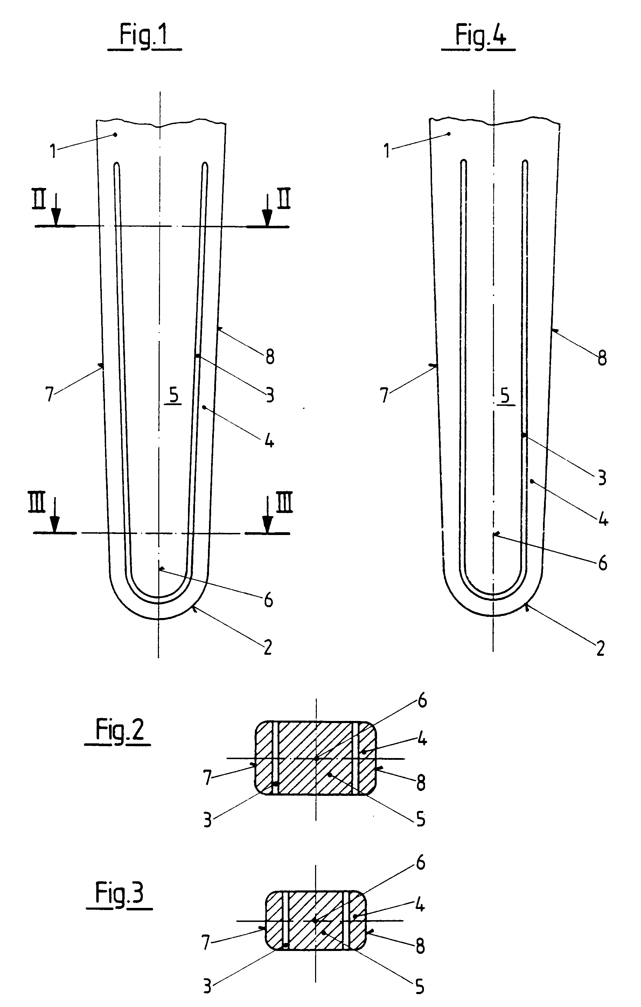

Der allseitig vom distalen Ende 2 her sich konisch erweiternde Schaft 1, von dem nur der distale Bereich dargestellt ist, ist mit einem zwischen 0,5 und 1 mm breiten Schlitz 3 versehen. Dieser durchsetzt die ganze Schaftdicke in Richtung ventral/ dorsal, wie die Fig. 2 und 3 zeigen. Dadurch entsteht ein am nicht dargestellten Knochen oder Zementbett anliegender Randbereich 4 und ein zungenartiger Kernbereich 5.

Bei Biegebelastungen auf den nicht dargestellten Gelenkkopf bewegt sich der Kernbereich in dem Schlitz 3 sowohl in Richtung lateral/distal - d.h. in der Zeichenebene der Figur horizontal - als auch in Richtung ventral/dorsal - d.h. in der Zeichenebene von Fig.2 und 3 vertikal -. Weiterhin hat der Kern noch die Möglichkeit einer Verdrehung um seine Längsachse 6.

Die Breite des U-förmigen Schlitzes 3 muss dabei zwei entgegengesetzten Anforderungen erfüllen:

Zum einen soll der Schlitz so schmal sein, dass in ihm kein Knochengewebe einwächst, was seine Breite auf etwa 1 mm begrenzt; zum anderen soll die Schlitzbreite so gross sein, dass der zungenartige Kern 5 bei den geschilderten Bewegungen den Schlitz nicht vollständig überbrückt und den Rand 4 berührt. Denn durch eine Berührung beider Teile 4 und 5 entsteht unerwünschter Metallabrieb. Die Länge der U-Schenkel des Schlitzes 3 in Richtung der Längsachse 6 sollte mehr als ein Drittel der Verankerungslänge des Schaftes im Knochen betragen, um die gewünschte Elastizität im Schaft zu erreichen.

Wie die Fig. 1 und 4 bis 6 verdeutlichen, können die Schenkel der U-Form parallel zur lateralen und medialen Aussenseite 7, 8 (Fig. 1) oder parallel zur Längsachse 6 (Fig. 4) verlaufen. Damit werden, wie bereits erwähnt, für den Kern 5 entweder eine über die ganze Schlitzhöhe konstante Steifigkeit oder Elastizität oder eine nach proximal zunehmende Steifigkeit erreicht.

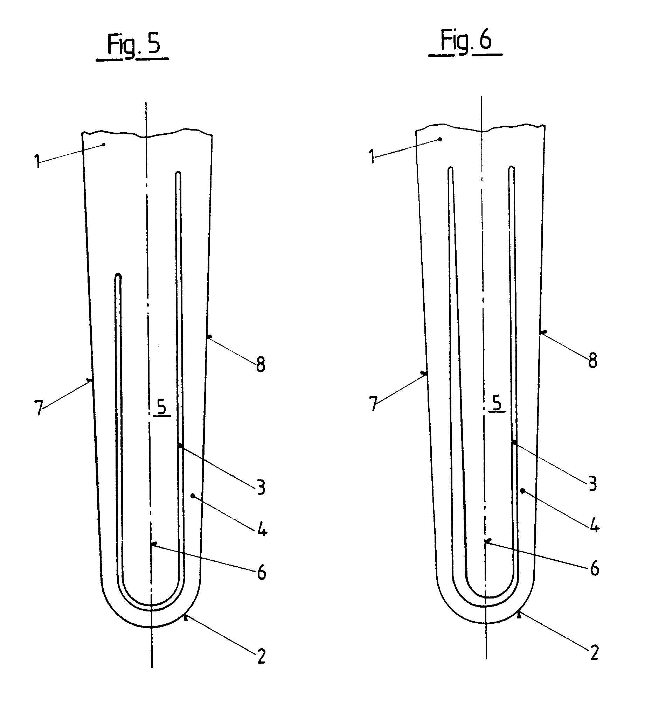

Unterschiedliche Steifigkeiten des Kerns lateral und medial der Längsachse 6 erhält man mit der Ausführungsform nach Fig. 5, bei der der laterale Schenkel der U-Form kürzer ist als der mediale.

Bei der Ausführungsform nach Fig. 6 erweitert sich der laterale Schenkel, dessen Breite am proximalen Ende gleich derjenigen des medialen ist, nach distal stetig. Seine Breite kann am distalen Ende bis zu etwa 4 mm betragen. Mit dieser Ausführungsform wird dem Kern 5 nach lateral eine grössere Bewegungsmöglichkeit eingeräumt als nach medial, da die Biegebelastungen nach lateral eine grössere Bewegungsamplitude erzeugen als nach medial. Ein Einwachsen von Knochengewebe in den verbreiterten Schlitz 3 ist nicht zu befürchten, weil die praktisch immer vorhandenen Bewegungen eine Bildung solchen Gewebes verhindern.

Das geeignetste Material für den neuen Schaft sind die in der Implantat-Technik gebräuchlichen Metalle oder Metall-Legierungen; bevorzugt werden dabei Titan oder eine Titan-Legierung.

Für die Herstellung des Schlitzes 3 hat sich vor allem das Hochdruck-Flüssigkeits-Strahl-Schneiden bewährt.

## Patentansprüche

1. Femurkopfprothese mit einem sich von distal nach proximal allseitig konisch erweiternden Verankerungsschaft (1), der mindestens einen die ganze Schaftdicke durchsetzenden, im wesentlichen in Längsrichtung verlaufenden schmalen Schlitz (3) aufweist, dadurch gekennzeichnet, dass der Schlitz (3) U-förmig im distalen Bereich des Schaftes (1) angeordnet ist, wobei die freien Enden der Schenkel des U-förmigen Schlitzes nach proximal gerichtet sind.

2. Prothese nach Anspruch 1, dadurch gekennzeichnet, dass sich die in Längsrichtung verlaufenden Schenkel des Schlitzes (3) parallel zur Aussenform (7, 8) des Schaftes (1) erstrecken.

3. Prothese nach Anspruch 1, dadurch gekennzeichnet, dass die in Längsrichtung verlaufenden Schenkel des Schlitzes (3) parallel zur Schaftachse (6) verlaufen.

4. Prothese nach Anspruch 3, dadurch gekennzeichnet, dass der lateral der Schaftachse (6) verlaufende Schenkel kürzer ist als der mediale Schenkel.

5. Prothese nach Anspruch 3 oder 4, dadurch gekennzeichnet, dass der laterale Schenkel breiter ist als der mediale, wobei seine Breite, ausgehend von einer gleichen Breite am proximalen Ende, von proximal nach distal stetig zunimmt.

6. Prothese nach Anspruch 1, dadurch gekennzeichnet, dass gemessen in der Richtung der Schaftachse (6) die Länge der Schenkel des Schlitzes (3) grösser als ein Drittel der Verankerungslänge des Schaftes im Knochen ist.

## Claims

1. A femoral head prosthesis having a fixing stem (1) which widens conically on all sides from the distal zone to the proximal zone and which is formed with at least one substantially longitudinal narrow slot (3) extending right through the thickness of the stem,
characterised in that the slot (3) is disposed in a U-shape in the distal zone of the stem (1), the free ends of the arms of the U-shaped slot being directed towards the proximal zone.

2. A prosthesis according to claim 1,
characterised in that the longitudinal arms of the slot (3) extend parallel to the external shape (7, 8) of the stem (1).

3. A prosthesis according to claim 1,
characterised in that the longitudinal arms of the slot (3) extend parallel to the stem axis (6).

4. A prosthesis according to claim 3,
characterised in that the arm which extends laterally of the stem axis (6) is shorter than the medial arm.

5. A prosthesis according to claim 3 or 4,
characterised in that the lateral arm is wider than the medial arm, its width, starting from the same width at the proximal end, increasing continuously from the proximal zone to the distal zone.

6. A prosthesis according to claim 1,
characterised in that the length of the arms of the slot (3) measured im the direction of the stem axis (6) is greater than one-third of the stem anchorage length in the bone.

## Revendications

1. Prothèse de tête de fémur comportant une tige d'ancrage (1) s'élargissant coniquement de tous côtés, de la partie distale vers la partie proximale, qui présente au moins une fente (3) étroite traversant toute l'épaisseur de la tige, s'étendant essentiellement dans la direction longitudinale, caractérisée en ce que la fente (3) est pratiquée en U dans la zone distale de la tige (1), les extrémités libres des branches de la fente en U étant orientées vers la zone proximale.

2. Prothèse selon la revendication 1, caractérisée en ce que les branches, s'étendant dans la direction longitudinale, de la fente (3) sont parallèles à la forme extérieure (7, 8) de la tige (1).

3. Prothèse selon la revendication 1, caractérisée en ce que les branches, s'étendant dans la direction longitudinale, de la fente (3) sont parallèles à l'axe (6) de la tige.

4. Prothèse selon la revendication 3, caractérisée en ce que la branche, s'étendant latéralement à l'axe (6) de la tige, est plus courte que la branche médiale.

5. Prothèse selon la revendication 3 ou 4, caractérisée en ce que la branche latérale est plus large que la branche médiale, sa largeur, partant d'une égale largeur à l'extrémité proximale, augmentant de manière continue de la zone proximale vers la zone distale.

6. Prothèse selon la revendication 1, caractérisée en ce que la longueur des branches de la fente (3), mesurée dans la direction de l'axe (6) de la tige, est supérieure à un tiers de la longueur d'ancrage de la tige dans l'os.
